(19) Europäisches Patentamt
European Patent Office
Office européen des brevets

(11) Numéro de publication : **0 097 547 B1**

(12) # FASCICULE DE BREVET EUROPÉEN

(45) Date de publication du fascicule du brevet : **28.08.85**

(51) Int. Cl.[4] : **C 07 C147/14**, A 61 K 31/16

(21) Numéro de dépôt : **83401049.8**

(22) Date de dépôt : **26.05.83**

(54) **Acides halogénobenzhydrylsulfinylacétohydroxamiques, procédé de préparation et utilisation en thérapeutique.**

(30) Priorité : **04.06.82 FR 8209805**

(43) Date de publication de la demande : **04.01.84 Bulletin 84/01**

(45) Mention de la délivrance du brevet : **28.08.85 Bulletin 85/35**

(84) Etats contractants désignés : **AT BE CH DE FR GB IT LI LU NL SE**

(56) Documents cités : **US-A- 4 066 686**

(73) Titulaire : **LABORATOIRE L. LAFON Société anonyme dite: 1 rue Georges Médéric F-94701 Maisons-Alfort (FR)**

(72) Inventeur : **Lafon, Louis 5 rue de l'Alboni F-75016 Paris (FR)**

(74) Mandataire : **Combe, André et al CABINET BEAU DE LOMENIE 55 rue d'Amsterdam F-75008 Paris (FR)**

**EP 0 097 547 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne, en tant que produits industriels, de nouveaux dérivés appartenant à la famille des acides benzhydrylsulfinylacétohydroxamiques et présentant au moins un groupe halogéné tel que F, Cl, Br ou $CF_3$ sur l'un des groupes phényle du reste benzhydryle. L'invention concerne également le procédé de préparation de ces nouveaux produits et leur utilisation en thérapeutique, notamment en tant que substances actives sur le système nerveux central (SNC).

On connaît déjà un brevet américain n° 4 066 686 un certain nombre d'acides benzhydrylsulfinylhydroxamiques de formule

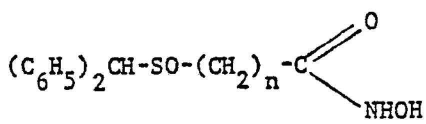

(Io)

(où n est 1, 2 ou 3). On sait également que les produits de formule Io sont des substances qui agissent sur le SNC. On sait en particulier que l'acide benzhydrylsulfinylacétohydroxamique (cf. exemple 1 du brevet précité ; n° de code : CRL 40028) est un excellent agent psychostimulant et que les acides benzhydrylsulfinylpropionhydroxamique (cf. exemple 6 du brevet précité ; n° de code : CRL 40260) et benzhydrylsulfinylbutyrohydroxamique (cf. exemple 9 du brevet précité ; n° de code : CRL 40278) sont des substances sédatives.

On vient de trouver de façon surprenante que les dérivés halogénés de formule I ci-après sont des substances (i) utiles en tant qu'agents psychostimulants, et (ii) douées de propriétés anti-agressives alors que le produit psychostimulant précité, l'acide benzhydrylsulfinylacétohydroxamique, ne présente pas de propriétés anti-agressives.

Les nouveaux dérivés d'acide benzhydrylsulfinylacétohydroxamique selon l'invention sont caractérisés en ce qu'ils sont choisis parmi l'ensemble constitué par les acides halogénobenzhydrylsulfinylacétohydroxamiques répondant à la formule générale

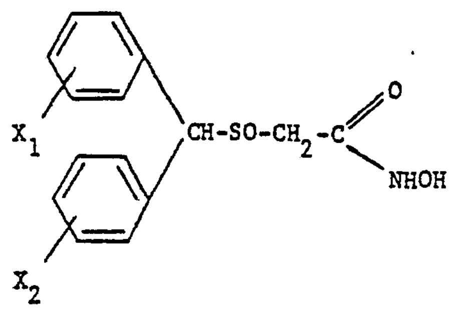

(I)

dans laquelle

— $X_1$ représente F, Cl, Br ou $CF_3$ ; et

— $X_2$, qui peut être identique ou différent de $X_1$, représente H, F, Cl, Br ou $CF_3$.

Les composés préférés selon l'invention sont ceux qui présentent dans leur formule développée le groupe $X_1$ en position para et le groupe $X_2$ = H ou $X_1$ également en position para. Parmi ceux-ci, on peut notamment mentionner les dérivés 4-chloro, 4-fluoro, 4,4'-dichloro et 4,4'-difluoro. Le produit le plus intéressant sur le plan thérapeutique est l'acide 4,4'-difluoro-benzhydrylsulfinylacétohydroxamique.

Pour illustration, le tableau I ci-après comporte quelques exemples de produits selon l'invention (exemple 1-exemple 8) et leurs homologues antérieurement connus du brevet américain précité (A1-A3).

Sur le plan neuropsychopharmacologique, les dérivés halogénés selon l'invention sont des agents psychostimulants. Comme le CRL 40028 (composé A1), ils augmentent la motilité spontanée chez la souris et améliorent la récupération motrice chez la souris dont la motilité a été réduite tant par habituation à l'enceinte que par agression hypoxique. En revanche, les CRL 40260 (composé A2) et CRL 40278 (composé A3) diminuent la motilité spontanée chez la souris et n'entraînent pas de récupération de l'activité motrice chez la souris dont la motilité a été réduite. La principale différence qui existe entre les dérivés halogénés selon l'invention et le psychostimulant (A1) réside dans le fait que lesdits dérivés halogénés ont des propriétés anti-agressives alors que le produit antérieurement connu ne présente pas de telles propriétés.

(voir tableau I page 3)

Tableau I

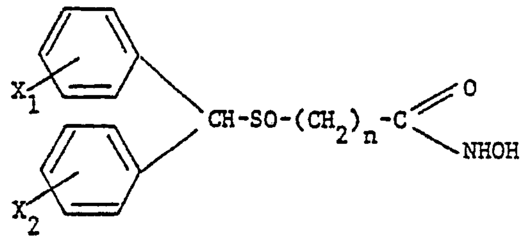

| Produit | n° de code | $X_1$ | $X_2$ | n | Effet sur le SNC |
|---|---|---|---|---|---|
| Exemple 1 | CRL 40941 | 4-F | 4-F | 1 | (b) |
| Exemple 2 | CRL 40933A | 4-Cl | H | 1 | (b) |
| Exemple 3 | CRL 41018A | 4-F | H | 1 | (b) |
| Exemple 4 | CRL 40933B | 4-Cl | 4-Cl | 1 | (b) |
| Exemple 5 | - | 4-Br | 4-Br | 1 | (b) |
| Exemple 6 | - | $4-CF_3$ | H | 1 | (b) |
| Exemple 7 | - | $4-CF_3$ | $4-CF_3$ | 1 | (b) |
| Exemple 8 | - | 4-Br | H | 1 | (b) |
| A1 (a) | CRL 40028 | H | H | 1 | (b) |
| A2 (a) | CRL 40260 | H | H | 2 | (c) |
| A3 (a) | CRL 40278 | H | H | 3 | (c) |

Notes :
(a) : décrit dans le brevet américain n° 4 066 686 ;
(b) : psychostimulant ;
(c) : sédatif.

On a résumé ci-après les essais comparatifs qui ont été entrepris en ce qui concerne les propriétés anti-agressives susvisées.

Le protocole opératoire est le suivant. Après avoir séjourné pendant 3 semaines dans chacune des moitiés d'une cage séparées par une cloison opaque, des groupes de trois souris mâles (quatre cages par produit et par dose) reçoivent les produits à tester par voie intrapéritonéale en suspension dans une solution aqueuse de gomme arabique, les animaux témoins (six cages) ne recevant que la solution aqueuse de gomme arabique. Une demi-heure plus tard, les deux groupes d'une même cage sont réunis par retrait de la cloison opaque et on note le nombre de combats qui surviennent en 10 minutes. Les résultats consignés dans le tableau II montrent que, même à la dose de 256 mg/kg qui provoque une excitation des souris, les dérivés halogénés de formule I inhibent nettement l'agressivité intergroupes en diminuant le nombre de combats par rapport aux témoins, ce que ne fait pas le psychostimulant A1.

Tableau II

| Produit | n° de code | Dose mg/kg | Variation de l'agressivité intergroupes |
|---|---|---|---|
| Exemple 1 | CRL 40941 | 256 (a) | – 20 % |
| Exemple 1 | CRL 40941 | 128 | – 61 % |
| Exemple 1 | CRL 40941 | 64 | – 58 % |

Tableau II (suite)

| | | | |
|---|---|---|---|
| Exemple 2 | CRL 40933A | 256 (a) | − 22 % |
| Exemple 2 | CRL 40933A | 128 | − 57 % |
| Exemple 2 | CRL 40933A | 64 | − 51 % |
| A1 | CRL 40028 | 256 (a) | + 36 % |
| A1 | CRL 40028 | 128 | − 3 % |
| A1 | CRL 40028 | 64 | + 2 % |

Note :
(a) : dose provoquant une excitation chez la souris.

Selon l'invention, on préconise une composition thérapeutique qui est caractérisée en ce qu'elle renferme, en association avec un excipient physiologiquement acceptable, au moins un composé de formule I en tant qu'ingrédient actif. Bien entendu, l'ingrédient actif sera utilisé à une dose pharmaceutiquement efficace.

Les dérivés halogénés selon l'invention peuvent être préparés selon une méthode connue en soi par application de mécanismes réactionnels classiques. Le procédé que l'on préconise comprend successivement :

a) la réaction d'un halogénobenzhydrylthioacétate d'alkyle de formule

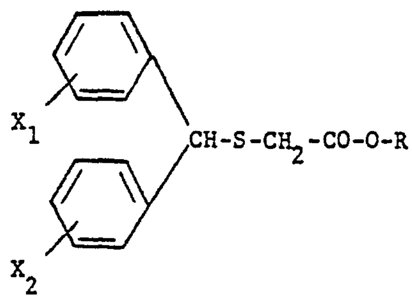

(II)

(où $X_1$ et $X_2$ sont définis comme ci-dessus et R représente un groupe alkyle en $C_1$-$C_2$) avec l'hydroxylamine en quantités stœchiométrique en présence de Na, NaOH ou KOH et au sein d'un alcool ROH (où R est défini comme ci-dessus), pour obtenir l'acide halogéno-benzhydrylthioacétohydroxamique correspondant de formule

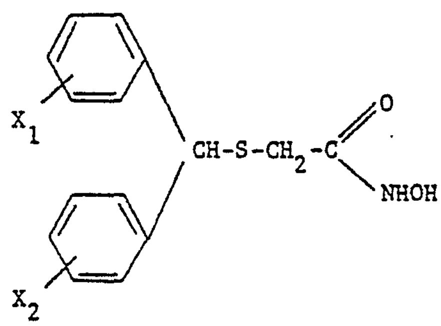

(III)

b) l'oxydation de l'acide III ainsi obtenu au moyen de $H_2O_2$ à 110-130 volumes, au sein d'acide acétique et à une température inférieure à 45 °C.

D'autres avantages et caractéristiques de l'invention seront mieux compris à la lecture qui va suivre d'exemples de préparation nullement limitatifs mais donnés à titre d'illustration.

Préparation I

Obtention de l'acide 4,4'-difluorobenzhydrylsulfinylacétohydroxamique.
(Exemple 1 ; n° de code : CRL 40941)

a) Acide 4,4'-difluorobenzhydrylthioacétohydroxamique.

14,7 g (0,05 mole) d'acide difluorobenzhydrylthioacétique (F. 117-118 °C) en solution dans 50 ml de

dichloroéthane sont traités avec 6 ml de méthanol et 0,5 ml d'acide sulfurique concentré. Après 6 h à reflux, on lave à l'eau, au bicarbonate dilué, sèche et évapore sous vide.

Le résidu huileux, qui comprend essentiellement le 4,4'-difluorobenzhydrylthioacétate de méthyle est traité pendant une nuit à 20 °C avec une solution obtenue avec 2,3 g (0,1 Atg) de sodium, 3,5 g (0,05 mole) de chlorhydrate d'hydroxylamine et 160 ml de méthanol anhydre.

On évapore à sec sous vide, reprend avec 200 ml d'eau, filtre sur charbon, acidifie avec HCl 3N, extrait à l'éther, lave à l'eau, sèche, évapore et cristallise le résidu d'évaporation dans l'éther de pétrole. On obtient l'acide hydroxamique attendu (F. = 75-76 °C) avec un rendement de 85 %.

b) CRL 40941

On oxyde 14,2 g (0,046 mole) d'acide 4,4'-difluorobenzhydrylthioacétohydroxamique en solution dans 50 ml d'acide acétique avec 4,6 ml d'eau oxygénée à 110 volumes. On évapore l'acide acétique sous vide, reprend à l'eau, essore et lave à l'eau. Par cristallisation dans le mélange méthanol-eau (50 : 50) v/v, on obtient le CRL 40941 avec un rendement global de 60 % (F. = 90-91 °C).

Préparation II

Obtention de l'acide 4-chlorobenzhydrylsulfinylacétohydroxamique.
(Exemple 2 ; n° de code : CRL 40933A)

En procédant comme indiqué à la préparation I, en remplaçant l'acide 4,4'-difluorobenzhydrylthioacétique par l'acide 4-chlorobenzhydrylthioacétique (F. 101-102 °C), on obtient le 4-chlorobenzhydrylthioacétate de méthyle (que l'on n'isole pas), l'acide 4-chlorobenzhydrylthioacétohydroxamique, puis, par oxydation au moyen de $H_2O_2$, le CRL 40933A.

On a résumé ci-après les résultats des essais réalisés avec le CRL 40941 (produit de l'exemple 1) en ce qui concerne ses propriétés neurosychopharmacologiques. Dans ces essais le CRL 40941 a été administré par voie intrapéritonéale, en suspension dans une solution aqueuse de gomme arabique, sous un volume de 20 ml/kg chez la souris mâle, et sous un volume de 5 ml/kg chez le rat mâle.

I. Toxicité

La DL-O (dose maximale non mortelle) du CRL 40941 par voie I. P. chez la souris mâle est supérieure à 512 mg/kg.

Le tiers des souris recevant 1 024 mg/kp I. P. de CRL 40941 présente des crampes abdominales, une respiration déprimée, et une sédation 45 min après administration, puis meurt 24 h après administration.

II. Comportement global et réactivités

Des lots de 3 animaux sont observés avant, puis 15 min, 30 min, 1 h, 2 h, 3 h, et 24 h après administration. On constate

1. Chez la souris

— à la dose de 256 mg/kg :
 une sédation fugace (d'une durée de 0,25 h), puis une excitation pendant 3 h,
 une hypothermie (— 1,9 °C) pendant 3 h, et
 une hypermotilité,
— à la dose de 64 mg/kg
 une sédation fugace durant moins de 0,25 h,
— à la dose de 16 mg/kg :
 une sédation fugace (durée inférieure à 0,25 h,

2. Chez le rat

— à la dose de 128 mg/kg :
 une excitation pendant la première heure qui suit l'administration,
 une augmentation de la réactivité au toucher pendant 1 à 3 h,
 une respiration déprimée pendant 0,5 h,
 une mydriase pendant plus de 3 h,
— à la dose de 32 mg/kg :
 une sédation de 0,5 h, et
 une respiration déprimée pendant 0,5 h.

III. Essais sur le SNC

A. Interaction avec l'apomorphine

1. Chez la souris

Des lots de 6 souris reçoivent le CRL 40941 une demi-heure avant l'injection sous-cutanée d'apomorphine, à la dose de 1 ou 16 mg/kg.

On observe que le CRL 40941 laisse inchangées l'hypothermie, l'attitude de verticalisation et les stéréotypies induites par l'apomorphine chez la souris.

2. Chez le rat

Le CRL 40941 est administré à des lots de 6 rats une demi-heure avant l'injection sous cutanée de 0,5 mg/kg d'apomorphine, On constate que notamment à la dose de 128 mg/kg, le CRL 40941 prolonge la durée des stéréotypies produites par l'apomorphine chez le rat, alors que le CRL 40028, antérieurement connu, ne modifie pas, à la dose de 256 mg/kg, le comportement stéréotypé induit par l'apomorphine chez le rat.

B. Interaction avec l'amphétamine

L'amphétamine (2 mg/kg) est injectée par voie intrapéritonéale à des lots de 6 rats, une demi-heure après l'administration de CRL 40941. On observe que, notamment à la dose de 128 mg/kg, le CRL 40941 potentialise en durée les stéréotypies amphétaminiques, alors que le CRL 40028 à la dose de 256 mg/kg ne modifie pas le comportement stéréotype dû à l'amphétamine.

C. Interaction avec la réserpine

Quatre heures après l'injection intrapéritonéale de 2,5 mg/kg de réserpine, des lots de 6 souris reçoivent le CRL 40941. On remarque que, à forte dose (128 mg/kg), le CRL 40941 diminue très modérément l'intensité du ptôsis réserpinique mais ne modifie pas l'hypothermie.

D. Interaction avec l'oxotrémorine

Le CRL 40941 est administré à des lots de 6 souris une demi-heure avant l'injection intrapéritonéale de 0,5 mg/kg d'oxotrémorine.

1. Action sur la température

A toutes les doses utilisées, le CRL 40941 s'oppose très partiellement à l'action hypothermisante de l'oxotrémorine, mais cet antégonisme très limité ne croît pas avec la dose.

2. Action sur les tremblements

Le CRL 40941 ne modifie pas de façon sensible les tremblements dus à l'oxotrémorine.

3. Action sur les symptômes cholinergiques périphériques

Le CRL 40941 laisse pratiquement inchangés les signes de stimulation cholinergique périphérique produits par l'oxotrémorine.

E. Action sur le test des quatre plaques, la traction et l'électrochoc

Le test est pratiqué sur les lots de 10 souris, une demi-heure après l'administration de CRL 40941.

L'on observe que le CRL 40941 n'entraîne pas d'augmentation du nombre de passages punis. Il ne provoque pas de déficit moteur majeur et, à la différence du CRL 40028 précité, il ne modifie pas les effets convulsivants et létaux de l'électrochoc.

F. Action sur la motilité spontanée

Une demi-heure après avoir reçu le CRL 40941, les souris (6 par dose, 12 témoins) sont placées en actimètre où leur motilité est enregistrée pendant 30 min.

Aux doses de 64 et 256 mg/kg, on observe que le CRL 40941 stimule l'activité motrice spontanée de la souris.

G. Action vis à vis de quelques comportements perturbés par divers agents

1. Motilité réduite par habituation à l'enceinte

Après 18 h de séjour dans les actimètres, les souris (6 par dose, 12 témoins) reçoivent le CRL 40941. Elles sont aussitôt replacées dans leurs enceintes respectives et, une demi-heure plus tard, on enregistre leur motilité pendant 30 min.

Aux doses de 64 et 256 mg/kg, le CRL 40941 provoque une reprise très nette de l'activité chez la souris habituée à son enceinte. A dose plus faible (4 et 16 mg/kg), cet effet est moins net.

2. Motilité réduite par agression hypoxique

Une demi-heure après avoir reçu le CRL 40941, les souris (10 par dose, 20 témoins) sont soumises à une anoxie hypobare aiguë [dépression de 600 mm Hg (c'est-à-dire environ $8 \times 10^4$ Pa) en 90 s, puis détente de 45 s], puis elles sont placées en actimètre où leur mobilité est enregistrée pendant 10 min.

Aux doses de 16, 64 et surtout 256 mg/kg, on observe que le CRL 40941 entraîne une amélioration de la récupération motrice chez la souris dont la motilité a été déprimée à la suite d'un séjour bref dans une enceinte à pression réduite.

3. Anoxie asphyxique

Des lots de 10 souris reçoivent le CRL 40941 une demi-heure avant l'administration intrapéritonéale de 32 mg/kg de triiodéthylate de gallamine.

A forte dose (256 mg/kg), le CRL 40941 raccourcit le délai d'apparition des convulsions et de la mort consécutives à une anoxie asphyxique provoquée par le triiodoéthylate de gallamine qui est un curarisant de référence.

H. Interaction avec le barbital

Une demi-heure après l'administration de CRL 40941, des lots de 10 souris reçoivent une injection intrapéritonéale de barbital (220 mg/kg).

Aux doses de 64 et 128 mg/kg, le CRL 40941 diminue nettement la durée du sommeil barbiturique.

I. Conclusion

Le CRL 40941 a des propriétés neurospychopharmacologiques analogues à celles du CRL 40028 qui est un agent psychostimulant connu. Il se distingue de ce dernier par ses propriétés anti-agressives et son action vis-à-vis de l'électrochoc.

On a également constaté que le CRL 40941 présente un autre avantage par rapport au CRL 40028 en ce qui concerne l'assimilation par voie orale ; en effet le CRL 40941 est en tant que psychostimulant nettement plus efficace par voie orale, chez le rat et chez l'homme, que le CRL 40028.

Du point de vue clinique, le CRL 40941 agit à des doses deux fois plus faibles que le CRL 40028. On a obtenu chez l'homme d'excellents résultats quand il était administré per os, à raison de 2 à 3 gélules ou comprimés (renfermant chacun 50 mg de principe actif) par jour, pendant notamment 2 à 8 semaines dans le traitement de l'hypersomnie et de la psychasténie.

**Revendications** (pour les Etats contractants : BE, CH, DE, FR, GB, IT, LI, LU, NL, SE)

1. Dérivé d'acide benzhydrylsulfinylacétohydroxamique, caractérisé en ce qu'il est choisi parmi l'ensemble constitué par les acides halogéno-benzhydrylsulfinylacétohydroxamiques répondant à la formule générale

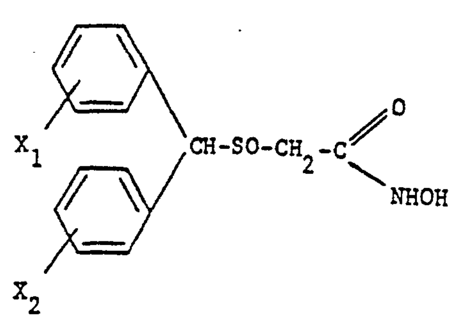

(I)

dans laquelle

— $X_1$ représente F, Cl, Br ou $CF_3$ ; et

— $X_2$, qui peut être identique ou différent de $X_1$, représente H, F, Cl, Br ou $CF_3$.

2. Dérivé selon la revendication 1, caractérisé en ce que les groupes $X_1$ et $X_2$ sont en position para.

3. Dérivé selon la revendication 1, caractérisé en ce que $X_1$ est 4-Cl ou 4-F et $X_2$ est H, 4-Cl ou 4-F.

4. Acide 4,4'-difluoro-benzhydrylsulfinylacétohydroxamique.

5. Acide 4-chloro-benzhydrylsulfinylacétohydroxamique.

6. Composition thérapeutique, caractérisée en ce qu'elle renferme, en association avec un excipient physiologiquement acceptable, au moins un acide halogéno-benzhydrylsulfinylacétohydroxamique selon l'une quelconque des revendications 1 à 5.

7. Procédé de préparation d'un acide halogéno-benzhydrylsulfinylacétohydroxamique selon la revendication 1, ledit procédé étant caractérisé en ce que successivement :

a) on fait réagir un halogénobenzhydrylthioacétate d'alkyle de formule

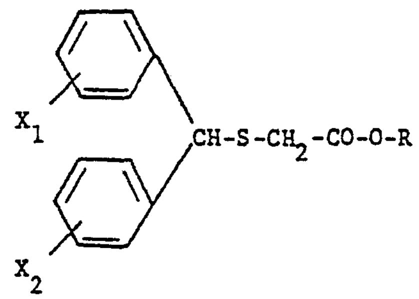

(II)

(où $X_1$ et $X_2$ sont définis comme ci-dessus et R représente un groupe alkyle en $C_1$-$C_2$) avec l'hydroxylamine en quantités stoechiométriques en présence de Na, NaOH ou KOH et au sein d'un alcool ROH (où R est défini comme ci-dessus), pour obtenir l'acide halogéno-benzhydrylthioacétohydroxamique correspondant de formule

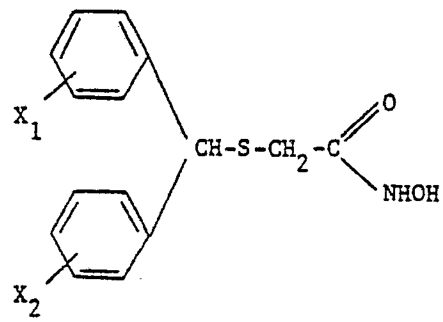

(III)

b) on soumet l'acide III ainsi obtenu à une réaction d'oxydation au moyen de $H_2O_2$ à 110-130 volumes, dans de l'acide acétique, à une température inférieure ou égale à 45 °C.

**Revendications** (pour l'Etat contractant AT)

1. Procédé de préparation d'un acide halogénobenzhydrylsulfinylacétohydroxamique de formule générale

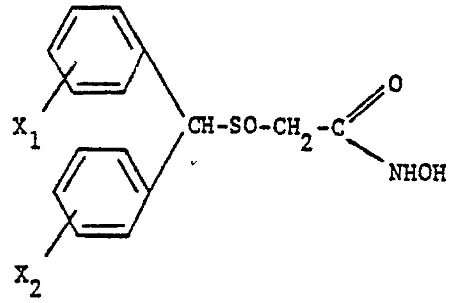

(I)

dans laquelle

— $X_1$ représente F, Cl, Br ou $CF_3$ ; et

— $X_2$, qui peut être identique ou différent de $X_1$, représente H, F, Cl, Br ou $CF_3$, ledit procédé étant caractérisé en ce que successivement :

a) on fait réagir un halogénobenzhydrylthioacétate d'alkyle de formule

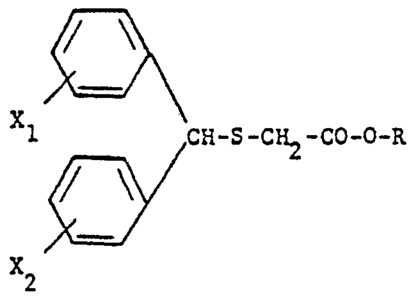

(II)

(où $X_1$ et $X_2$ sont définis comme ci-dessus et R représente un groupe alkyle en $C_1$-$C_2$) avec l'hydroxylamine en quantités stoechiométriques en présence de Na, NaOH ou KOH et au sein d'un alcool ROH (où R est défini comme ci-dessus), pour obtenir l'acide halogéno-benzhydrylthioacétohydroxamique correspondant de formule

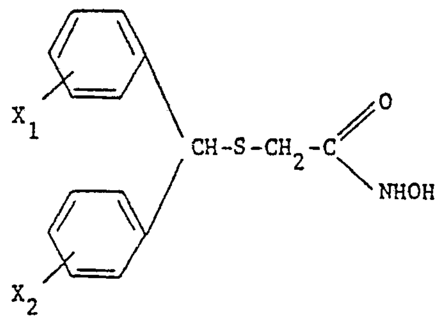

(III)

b) on soumet l'acide III ainsi obtenu à une réaction d'oxydation au moyen de $H_2O_2$ à 110-130 volumes, dans de l'acide acétique, à une température inférieure ou égale à 45 °C.

2. Procédé selon la revendication 1, caractérisé en ce que les groupes $X_1$ et $X_2$ sont en position para.
3. Procédé selon la revendication 1, caractérisé en ce que $X_1$ est 4-Cl ou 4-F et $X_2$ est H, 4-Cl ou 4-F.
4. Procédé selon la revendication 1, caractérisé en ce que $X_1$ est 4-F et $X_2$ est 4-F.
5. Procédé selon la revendication 1, caractérisé en ce que $X_1$ est 4-Cl et $X_2$ est H.
6. Utilisation des produits tels qu'obtenus selon l'une des revendications précédentes et ayant pour formule (I) pour la préparation de compositions thérapeutiques actives comme agents psychostimulants doués des propriétés anti-agressives.

**Claims** (for the Contracting States : BE, CH, DE, FR, GB, IT, LI, LU, NL, SE)

1. A benzhydrylsulfinylacetohydroxamic acid derivative characterized in that it is selected from the group consisting of the halogeno-benzhydrylsulfinylacetohydroxamic acids of the formula

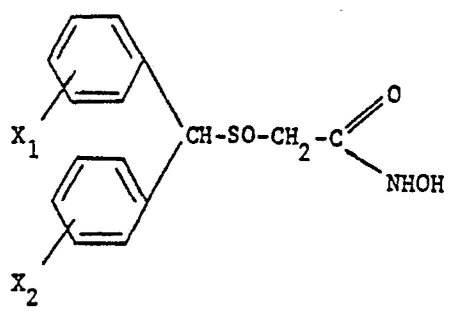

(I)

wherein

— $X_1$ is F, Cl, Br or $CF_3$ ; and

— $X_2$, which may be identical to or different from $X_1$, is H, F, Cl, Br or $CF_3$.

2. A derivative according to claim 1, characterized in that $X_1$ and $X_2$ are in para position.
3. A derivative according to claim 1, characterized in that $X_1$ is 4-Cl or 4-F, and $X_2$ is H, 4-Cl or 4-F.

4. 4,4'-Difluoro-benzhydrylsulfinylacetohydroxamic acid.

5. 4-Chloro-benzhydrylsulfinylacetohydroxamic acid.

6. A therapeutical composition characterized in that it comprises, in association with a physiologically acceptable excipient, at least one halogeno-benzhydrylsulfinylacetohydroxamic acid according to any one of claims 1 to 5.

7. A method for preparing a halogeno-benzhydrylsulfinyl-acetohydroxamic acid of formula I according to claim 1, characterized in that it successively comprises :

a) reacting stoichiometrically an alkyl halogenobenzhydrylthioacetate of the formula

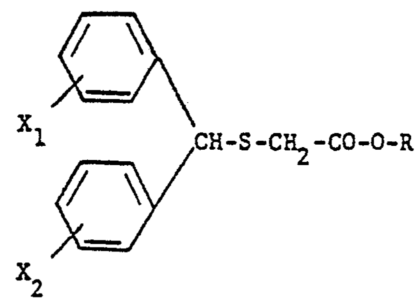

(II)

(wherein $X_1$ and $X_2$ are defined as indicated above, and R represents a $C_1$-$C_2$-alkyl group) with hydroxylamine in an alcohol ROH (wherein R is defined as indicated above) in the presence of a product selected from the group consiting of Na, NaOH and KOH, for obtaining the corresponding halogenobenzhydrylthioacetohydroxamic acid of the formula

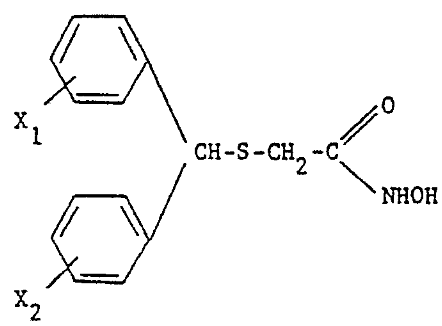

(III)

and

b) submitting the acid III thus obtained to an oxidation reaction with $H_2O_2$ at 110-130 volumes, in acetic acid, at a temperature lower than or equal to 45 °C.

**Claims** (for the Contracting State AT)

1. Process for the preparation of halogeno-benzhydrylsulfinylacetohydroxamic acid of formula

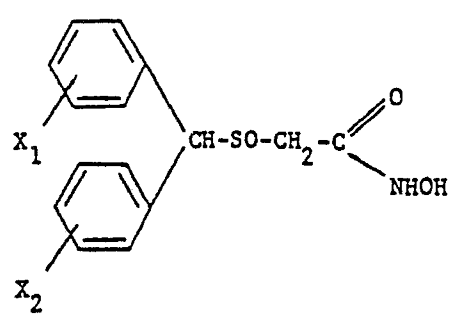

(I)

wherein

— $X_1$ is F, Cl, Br or $CF_3$ ; and

— $X_2$, which may be identical to or different from $X_1$, is H, F, Cl, Br or $CF_3$, said process being characterized in that it successively comprises :

a) reacting stoichiometrically an alkyl halogenobenzhydrylthioacetate of the formula

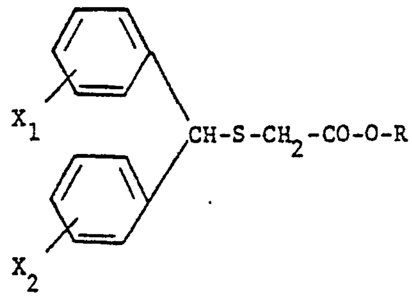

(II)

(wherein $X_1$ and $X_2$ are defined as indicated above, and R represents a $C_1$-$C_2$-alkyl group) with hydroxylamine in an alcohol ROH (wherein R is defined as indicated above) in the presence of a product selected from the group consisting of Na, NaOH and KOH, for obtaining the corresponding halogenobenzhydrylthioacetohydroxamic acid of the formula

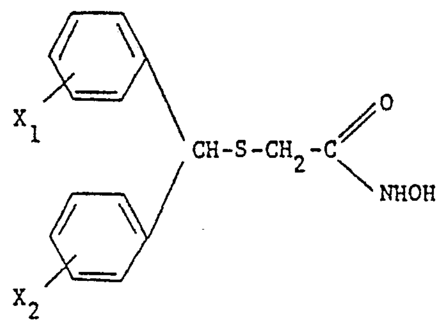

(III)

and

b) submitting the acid III thus obtained to an oxidation reaction with $H_2O_2$ at 110-130 volumes, in acetic acid, at a temperature lower than or equal to 45 °C.

2. Process according to claim 1, characterized in that $X_1$ and $X_2$ are in para position.

3. Process according to claim 1, characterized in that $X_1$ is 4-Cl or 4-F, and $X_2$ is H, 4-Cl or 4-F.

4. Process according to claim 1, characterized in that $X_1$ is 4-F and $X_2$ is 4-F.

5. Process accordint to claim 1, characterized in that $X_1$ is 4-Cl and $X_2$ is H.

6. Use of the products such as obtained according to any one of the preceding claims, and having the formula (I) for the preparation of active therapeutical compositions as psychostimulating agents having antiaggressive properties.

**Patentansprüche** (für die Vertragsstaaten : BE, CH, DE, FR, GB, IT, LI, LU, NL, SE)

1. Benzhydrylsulfinylacetohydroxsmsäure-Derivat, dadurch gekennzeichnet, daß es ausgewählt ist aus der Gruppe bestehend aus den Halogen-benzhydrylsulfinylacetohydroxamsäuren der allgemeinen Formel

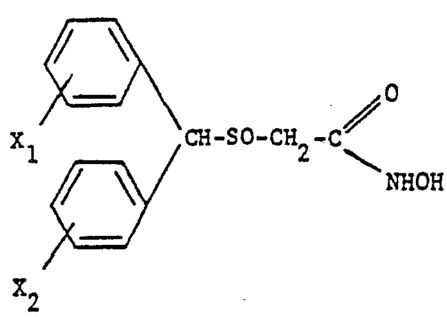

(I)

worin

— $X_1$ für F, Cl, Br oder $CF_3$ steht ; und

— $X_2$, das gleich oder unterschiedlich von $X_1$ sein kann, H, F, Cl, Br oder $CF_3$ bedeutet.

2. Derivat nach Anspruch 1, dadurch gekennzeichnet, daß die Gruppen $X_1$ und $X_2$ in para-Position stehen.

3. Derivat nach Anspruch 1, dadurch gekennzeichnet, daß $X_1$ für 4-Cl oder 4-F und $X_2$ für H, 4-Cl oder 4-F steht.

4. 4,4'-Difluor-benzhydrylsulfinylacetohydroxamsäure.

5. 4-Chlor-benzhydrylsulfinylacetohydroxamsäure.

6. Therapeutische Zusammensetzung, dadurch gekennzeichnet, daß sie in Verbindung mit einem physiologisch akzeptablen Exzipienten mindestens eine Halogen-benzhydrylsulfinylacetohydroxamsäure nach einem der Ansprüche 1 bis 5 enthält.

7. Verfahren zur Herstellung einer Halogen-benzhydrylsulfinylacetohydroxamsäure nach Anspruch 1, dadurch gekennzeichnet, daß nacheinander

a) ein Alkyl-halogenbenzhydrylthioacetat der Formel

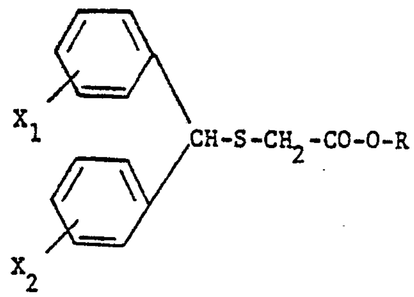

(II)

(worin $X_1$ und $X_2$ obige Bedeutung haben und R eine $C_1$-$C_2$-Alkylgruppe darstellt) mit Hydroxylamin in stöchiometrischen Mengen in Gegenwart von Na, NaOH oder KOH und in einem Alkohol ROH (worin R obige Bedeutung hat) umgesetzt wird, um die Halogen-benzhydrylthioacetohydroxamsäure der Formel

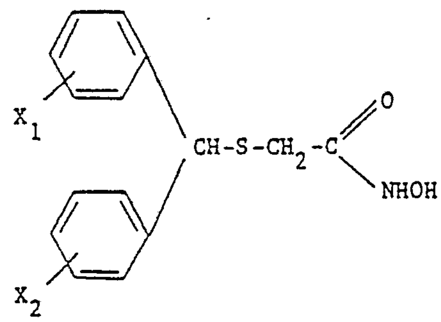

(III)

zu erhalten,

b) die so erhaltene Säure III einer Oxidationsreaktion mittel $H_2O_2$ von 110 bis 130 Volumsteilen in Essigsäure bei einer Temperatur von unter oder gleich 45 °C unterzogen wird.

**Patentansprüche** (für den Vertragsstaat AT)

1. Verfahren zur Herstellung Halogen-benzhydrylsulfinylacetohydroxamsäuren der allgemeinen Formel

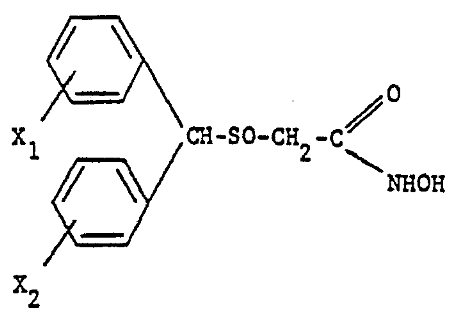

(I)

worin

— $X_1$ für F, Cl, Br oder $CF_3$ steht ; und

— $X_2$, das gleich oder unterschiedlich von $X_1$ sein kann, H, F, Cl, Br oder $CF_3$ bedeutet, dadurch gekennzeichnet, daß nacheinander

a) ein Alkyl-halogenbenzhydrylthioacetat der allgemeinen Formel

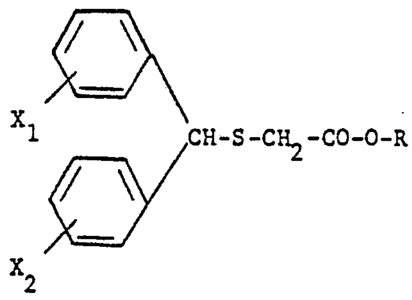

(II)

worin $X_1$ und $X_2$ obige Bedeutung haben und R eine $C_1$-$C_2$-Alkylgruppe darstellt, mit Hydroxylamin in stöchiometrischen Mengen in Gegenwart von Na, NaOH oder KOH und in einem Alkohol der allgemeinen Formel ROH, worin R obige Bedeutung hat, umgesetzt wird, um eine Halogen-benzhydrylthioacetohydroxamsäure der allgemeinen Formel

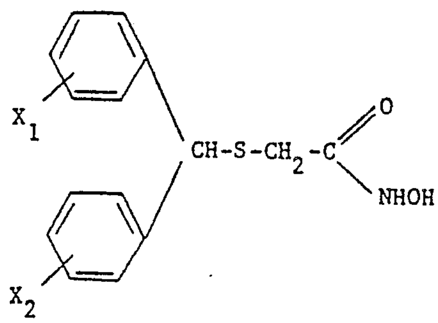

(III)

worin $X_1$ und $X_2$ wie obendefiniert sind, zu erhalten,

b) eine so erhaltene Säure der Formel (III) einer Oxidationsreaktion mittels $H_2O_2$ von 110 bis 130 Volumsteilen in Essigsäure bei einer Temperatur von unter oder gleich 45 °C unterzogen wird.

2. Verfahren nach Anspruch 1, dadurch gekennzeichnet, daß ein Alkyl-halogenbenzhydrylthioacetat der Formel (II), worin $X_1$, $X_2$ und R wie in Anspruch 1 definiert sind, mit der Maßgabe, daß $X_1$ in para-Stellung steht und $X_2$ in der Bedeutung F, Cl, Br und $CF_3$ in para-Stellung steht, eingesetzt wird.

3. Verfahren nach den Ansprüchen 1 und 2, dadurch gekennzeichnet, daß ein Alkyl-halogenbenzhydrylthoacetat der Formel (II), worin R wie in Anspruch 1 definiert ist, $X_1$ für 4-Cl oder 4-F und $X_2$ für H, 4-Cl oder 4-F steht, eingesetzt wird.

4. Verfahren nach den Ansprüchen 1 bis 3 zur Herstellung von 4,4'-Difluor-benzhydrylsulfinylacetohydroxamsäure, dadurch gekennzeichnet,daß ein Alkyl-4-4'-difluorbenzhydrylthioacetat der Formel (II), worin R die im Anspruch 1 angegebene Bedeutung hat, eingesetzt wird.

5. Verfahren nach den Ansprüchen 1 bis 3 zur herstellung von 4-Chlor-benzhydrylsulfinylacetohydroxamsäure, dadurch gekennzeichnet, daß ein Alkyl-4-chlorbenzhydrylthioacetat der Formel (II), worin R die im Anspruch 1 angegebene Bedeutung hat, eingesetzt wird.

6. Verwendung von nach den Ansprüchen 1 bis 5 hergestellten Halogen-benzhydrylsulfinylacetohydroxamsäuren der Formel (I) zur Herstellung von auf das Zentralnervensystem (ZNS) wirkenden therapeutischen Zusammensetzungen, welche in Verbindung mit physiologisch alzeptablen Exzipienten wenigstens eine substituierte Hydroxamsäure der Formel (I) enthalten.